# EUROPEAN PATENT SPECIFICATION

(11) **EP 3 331 479 B1**
(45) Date of publication and mention of the grant of the patent: **05.05.2021**
(21) Application number: 16741044.8
(22) Date of filing: 21.07.2016
(51) Int. Cl.: A61F 9/00, A61M 11/00

(54) **A DEVICE FOR APPLYING AN OPHTHALMIC FLUID**
EINRICHTUNG ZUR ANWENDUNG EINES OPHTHALMISCHEN FLUIDS
DISPOSITIF D'APPLICATION DE FLUIDE OPHTHALMIQUE

(30) Priority: 04.08.2015 DK 201570504
(43) Date of publication of application: 13.06.2018
(73) Proprietor: Eye-Go A/S, 3060 Espergærde (DK)
(72) Inventor: NIELSEN, Søren Anker, 3060 Espergærde (DK); SOLGÅRD, Kurt, 3230 Græsted (DK)
(74) Representative: Zacco Denmark A/S
(86) International application number: PCT/EP2016/067414
(87) International publication number: WO 2017/021168

(56) References cited:
- WO-A1-2015/114139
- US-A- 3 934 585
- US-A- 5 997 518

## Description

The present invention relates generally to a device for dispensing a mist of an ophthalmic liquid fluid. Such devices are already known and vary in structure and design. By way of example, devices are known that comprise a squeeze bottle squeezed by the user to expel the liquid fluid as a mist.

US patent 3,934,585 A shows a method and apparatus for applying therapeutic eye drops to the eye by metering a predetermined volume of fluid and rapidly applying a pressure to one end of the metered fluid for forcing the fluid from a nozzle.

US patent application 2002/161344 A shows a device and use method for ejecting a liquid stream towards an eye. The device includes a pump mechanism operable to deliver at least part of the liquid from a container through the opening to form a stream of liquid. The pump mechanism may include a pump driver able to store cocked energy for driving the pump mechanism and at least one activation mechanism may be present and operable to initiate the device for the liquid delivery, whereby a driving mechanism is arranged to transform manual or stored energy into cocked energy.

US patent 5,997,518 shows a device and method for delivering small microliter volumes of liquid preparations to the eye or other body part. The device and method introduces a volume of treatment liquid into an air stream and delivers the liquid in the form of small droplets to the desired site. In contrast to US patent 5,997,518 A the device according to the present invention includes a dosing chamber, for delivering doses of the liquid fluid to a fluid chamber from which a single dose is dispensed by the action of air expelled from an air chamber.

The present invention seeks to overcome problems with the prior art dispensers, without compromising the need to provide a simple and user friendly dispenser which allows for repeated discharge of a dose of a medical ophthalmic fluid. In particular, the present invention provides a device which is simple to use in allowing a ready dispensing for treatment of both eyes of a person. At the same time, through the invention it becomes possible to dispense the ophthalmic liquid fluid irrespectively of the user holding his head upright or tilted, or even if the user is in bed lying down.

Specifically, according to the first aspect as defined broadly in claim 1 device comprises a discharge opening for said mist, a mixing chamber communicating with a discharge opening, an air chamber, a first drive operable to expel air from the air chamber, a fluid chamber for holding a first volume of the liquid and communicating with the mixing chamber, a dosing chamber for containing a second volume of the liquid corresponding to a plurality of the doses, preferably to two doses, of the liquid, the dosing chamber communicating with or configured for communicating with a supply of the liquid, such as a collapsible liquid container or a liquid container having a movable bottom, and with the fluid chamber, a second drive operable to supply the second volume of the liquid from the supply to the dosing chamber and for emptying the dosing chamber by repetitive delivery of a single dose of the plurality of doses of the liquid contained therein to the fluid chamber, the air chamber communicating with the mixing chamber and/or with the fluid chamber for the expelled air to drive the liquid delivered to the fluid chamber into the mixing chamber by the expelled air.

The particular embodiment defined in claim 2 allows the user to obtain a very fine mist of the fluid required where the fluid has a high viscosity and concentration, as may be the case in certain applications and for treating certain ophthalmic diseases or conditions, such as dry eyes, requiring the deposition of low to high viscosity fluid on a person's cornea. Thus, fine, or relatively fine, droplets are achieved even where a liquid fluid of medium viscosity, such as in the order of 1-100 mPa.s is dispensed, through the action of a dedicated air flow, while according to another aspect a repeated discharge of identical doses is made possible.

It is also contemplated to include a mechanism allowing for a user to bring the device into a configuration ready for dispensing using little or a minimum of manual force, which may be required where to device is to be used by eg. an elderly person. For that purpose the device may further comprise a rotatable head or handle, the pistons of the device being moved by rotation of the handle.

Embodiments of the present invention are defined in the dependent claims, the objects of which will appear from the following.

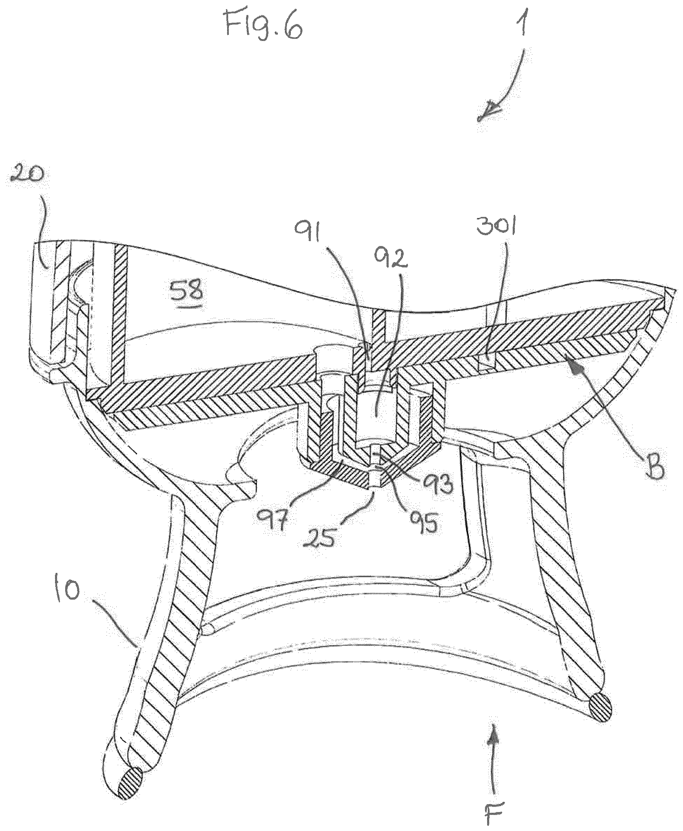

A presently preferred embodiment of the invention will now be described with reference to fig. 6 and onwards of the appended drawings; the following description is not intended to limit the scope of the present invention which is defined by the claims.
Fig. 1 is a side view of a prior device which is not in the public domain for dispensing a mist of an ophthalmic liquid fluid, which device is not as defined in the appended claims,
Fig. 2 is a perspective view of a component structure of the device of fig. 1,
Fig. 3 is a perspective view of the device of fig. 1, with an end cap and shown with the device casing partially cut away,
Fig. 4 is a cross-sectional view of the device of fig. 1 in a configuration prepared for dispensing a mist of fluid, and
Fig. 5 is another cross-sectional view of the device of fig. 1, perpendicular to that of fig. 4 and in a configuration after dispensing of a mist of fluid.
Fig. 6 is a perspective cross-sectional bottom view of an embodiment of the device of the invention, as claimed herein,
Fig. 7 is another perspective partial and cross-sectional view of the device of fig. 6, without the eyelid opener structure,
Fig. 8a and 8b are perspective views of the component parts of the bottom structure of the device of fig. 7 and 8, fig. 8a showing a component in an upside down position,
Fig. 9 is a cross-sectional view of the device of fig. 6 and 7, through the liquid supply and dosing chamber, and
Figs. 10a-10e show the device of figs. 6 and 7 in various configurations.

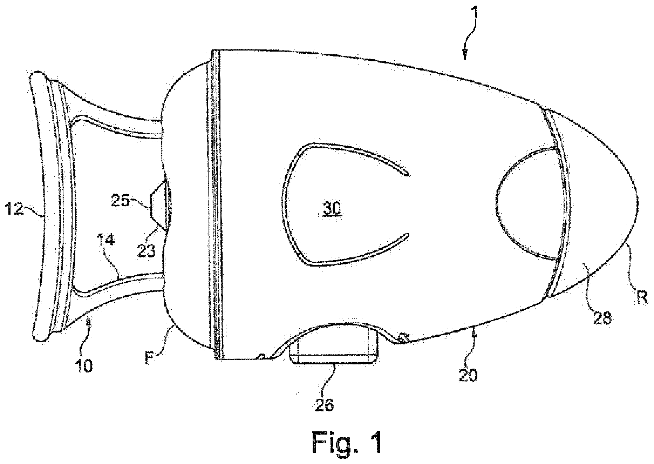

Figs. 1-5 show a prior device 1 (not publicly available) having certain fundamental structural features in common with the device according to the present invention, as well as features upon which the present invention does not rely.

When later herein discussing the present invention reference will be made to any common or similar features shown in figs. 1-5 using the same reference numerals and nomenclature. Additional structural features specific to the present invention, such as in particular the claimed dosing chamber and specially configured second drive, which are not shown in figs. 1-5, will be discussed and particularly pointed out with reference to the embodiment of the present invention shown in figs. 6 and onwards.

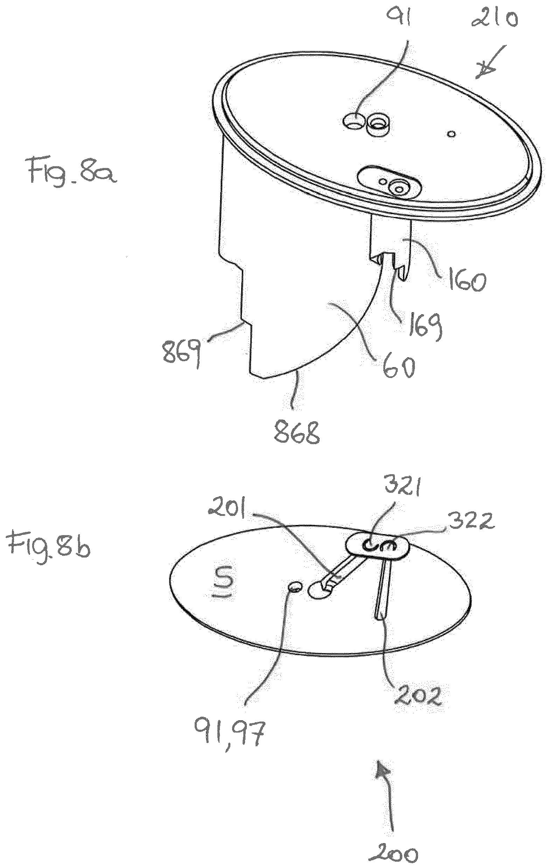

Certain features discussed with reference to fig. 6 and onwards, in particular as shown in figs. 8a and 8b, may be incorporated in the prior device 1 of figs. 1-5, and advantageously used therewith irrespectively of being shown herein in the context of the device of fig. 6.

Like the prior device shown in figs. 1-5 the device 1 of the presently claimed invention is for dispensing of a mist of an ophthalmic liquid fluid into a person's eye/onto a person's cornea, and is particularly but not exclusively useful for dispensing a mist of such a fluid having a relatively high viscosity. The device of the present invention is preferably sized to allow an average person to hold it in his/her hand, and is suitable for use irrespectively of the position in which it is held, such as whether held in a vertical or horizontal position.

The device 1 of the present invention may as shown in fig. 6 have essentially the same overall visual appearance as the device of fig. 1 and comprises an eyelid opener portion 10 and a casing portion 20. The eyelid opener portion 10 seen best in fig. 1 is configured for keeping a person's eyelid open by being held against the eye region during the dispensing and includes a collar 12 and a connecting structure 14 connecting the collar 12 with a front end F of the casing 20. The connection to the casing 20 may be so as to allow for replacement of the eyelid opener portion 10 with a smaller one, such as where the device 1 is to be used by a child. A discharge opening or nozzle 25, which may by way of example be circular or oval, for dispensing the aforementioned mist in the direction towards the collar 12 and, hence, the eye, is located at the front end F of the device 1.

The prior device 1 shown in figs. 1-5 generally includes various mechanisms or drives located inside the casing 20 and operable by a person through various operating parts which may include, as shown by way of example, one or more of following: a rotatable part 26, a depressible tab 30, and a rotatable handle or head 28 defining the rear end R of the casing 20.

In the device 1 according to the present invention, as discussed later with reference to fig. 6 and onwards, all drives are preferably operable by the user manipulating only the rotatable head 28 and the depressible tab 30, the latter defining a release part of a hold and release mechanism. This allows for an extremely simple use of the device 1, making it highly suitable also for elderly people.

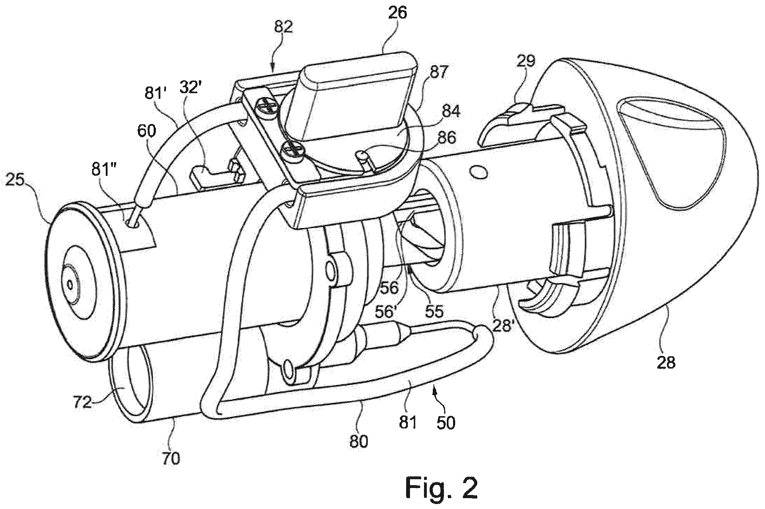

Turning to the prior device, fig. 2 shows an internal component structure generally designated numeral 50 and mounted inside the casing 20. The component structure 50 supports at one end an outlet structure including the discharge opening 25 and at the other end the rotatable head 28. The rotatable head 28 has a tubular extension 28' and is configured to snap into engagement with the casing 20 via flexible tabs 29, for assisting in the assembly of the device 1.

The component structure 50 comprises inter alia a container or barrel 70 containing the ophthalmic liquid fluid to be dispensed as a mist, and a first drive for user controlled advancing of a piston 55 with a piston rod and a head (the head not being visible in fig. 2) received by a piston cylinder 60.

The cylinder 60 is configured for storing a first volume of air in an internal air chamber when the piston 55 is in a first, normally fully retracted position. The barrel 70 preferably has a movable bottom 72 and/or may be collapsible, has an internal volume allowing for dispensing of multiple doses of the ophthalmic fluid, and may be replaceable to allow for replacement with another and, hence, continued use of the device 1 after one barrel 70 has been emptied.

For the reason explained below, in the prior device 1 the rod of the piston 55 is preferably held against rotation about its longitudinal axis and is generally movable to its first, retracted position from a second, advanced position shown in fig. 2 by rotation of the rotatable device head 28 about an axis parallel with the longitudinal axis of the rod of the piston 55.

In the prior device 1 shown in figs. 1-5 a proximal end part of the rod of the piston 55 remote from the head is shown in fig. 2 and is configured to be receivable within the tubular extension 28' of the rotatable device head 28. The rod of the piston 55 has surface track 56 including a spirally winding portion as well as a straight portion 56'. The winding portion of the surface track 56 slidably receives a tab located on the inside of the tubular extension 28' whereby rotation of the rotatable head 28 relative to the piston rod brings about a corresponding displacement of the piston 55 from the second position to the retracted first position in which a length of the proximal end part of the rod of the piston 55 shown in fig. 2 is received within the tubular extension 28' of the rotatable head 28.

Specific for the prior device 1 of figs. 1-5 is that the component structure 50 also may include another drive, shown and exemplified as a peristaltic dosing pump 82, for dispensing a required dose of the liquid fluid into a liquid chamber and/or a mixing chamber to be discussed further below and located at the fore end of the cylinder 60, inside thereof and closest to the discharge opening 25. Shown in fig. 2 is also a tube 80, preferably a tube flexible along its entire length, which has a first portion 81 and a second portion 81' and which is used for drawing the liquid fluid from the barrel 70. The tube 80 connects the barrel 70 with the aforementioned mixing chamber at a connection point 81", via the peristaltic dosing pump 82 discussed further below.

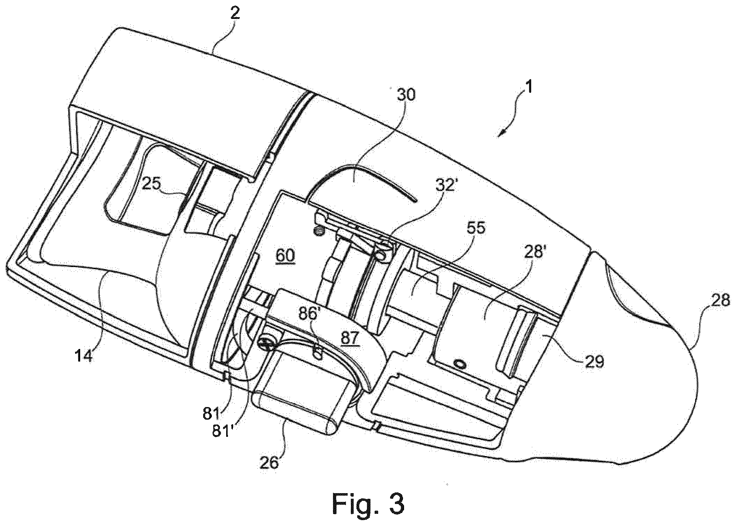

The peristaltic pump 82 includes a housing with a peripheral wall portion 87, shown as a semi-circular wall portion 87, as well as a rotatable disc 84 carrying on the one hand a handle 26 for manually operating the pump 82 and on the other hand two radially oppositely lodged shoes or rollers, of which one roller 86 is seen best in fig. 2 while the opposite roller 86' is seen in fig. 3. A flexible portion of the tube 80 is received between the wall portion 87 and the rotatable disc 84 and is locally squeezed flat between the wall portion 87 and each of the two rollers 86. The length of the tube 80 between the locally flattened parts thereof defines a volume or dose of the fluid inside that length which is driven forward into the second portion 81' of the tube 80 by the user rotating the disc 84 and, hence, moving the rollers 86, 86' along the wall portion 87 and the tube portion between disc 84 and the wall portion 87. A mechanism (not shown) may be included for limiting rotation of the rotatable disc 84 by eg. 180° per operation, such that manipulation of the handle 26 only allows the user to draw a single dose of predetermined volume of the liquid fluid from the barrel 70 until the time that mechanism is released. It will be understood that proper configuration of the tube 80 and the container 70 ensures that a corresponding volume of the liquid fluid in the container 70 is drawn from the container 70 as the dose is driven forward into the second portion 81' of the tube 80 in the manner described above. By using a collapsible container or reservoir 70, or a container 70 with a displaceable bottom, suction applied by the peristaltic pump 82 makes it possible to ensure a complete emptying of the container 70.

The peristaltic pump 82 may be provided with, integrally or not, not shown valve means configured for hindering flow of liquid fluid when the peristaltic pump 82 is passive.

The prior device 1 may be configured such that the first drive and the peristaltic pump 82 may be activated and/or retracted by means of one button or handle only; eg. the device head 28 and the handle 26. Furthermore, the device 1 may be configured such that first and/or the peristaltic pump 82 may be activated and/or retracted and/or released by means of applying a linear force to a pushbutton. Fig. 3 shows the device 1 with a protective cap 2 mounted onto the front end F.

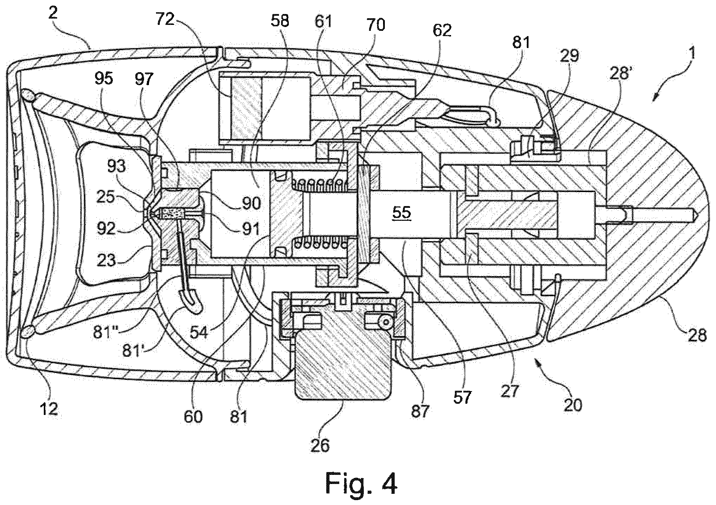

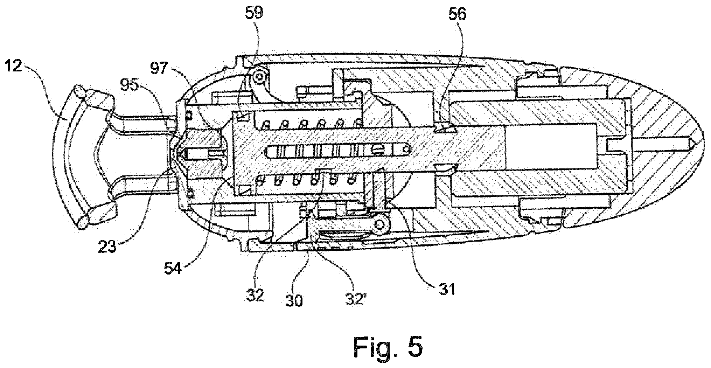

Turning to figs. 4 and 5, further details of the prior device 1 shown in figs. 1-3 and of the present device 1 will now be discussed.

Fig. 4 shows the device 1 in the first, retracted position of the piston 55, i.e. where the head 54 of the piston is positioned distant from the discharge opening 25 and where a relatively large length of the proximal end part of the rod 57 of the piston 55 is received within the tubular extension 28' of the rotatable head 28. The user may displace the piston 55 relative to the cylinder 60 to this first position by rotating the head 28; this displacement is against the force of a spring 61 configured to bias the piston 55 towards the aforementioned second, advanced position by being compressed between the piston head 54 and a rear closure 62 of the cylinder 60. Hence, the head 28 defines an energizer in that rotation of the head 28 energized the spring 61 through the compression thereof. In the prior device 1, the aforementioned tab located inside the tubular extension 28' and engaging the spirally winding portion of surface track 56 is shown in fig. 4 by numeral 27, and by selecting an appropriate design of the spirally winding portion of the surface track 56 a desired displacement of the piston 55 is achieved by rotating the head 28 through eg. 180° or 360°, thus allowing also users with reduced manual strength to retract the piston 55 against the force of the spring 61, with a relatively small effort.

In the first position of the piston 55 of the prior device 1 shown in fig. 4 the tab 27 is aligned with the straight portion 56' of the surface track 56, the piston 55 being ready for moving axially along the length of the extension 28' to its second position, driven by the spring 61 upon the user releasing a lock mechanism. The lock mechanism comprises a locking rib 31 connected to the depressible tab 30 (see fig. 1) and engaging a recess 32 formed in the outer face of the cylinder 60. The rib 31 and the recess 32 are best seen in fig. 5. The depressible tab 30 acts as a lever mechanism by including a depressible arm 32' also shown in fig. 2, whereby depression of the tab 30 draws the locking rib 31 located at the other end of arm 32' out of engagement with the recess 32 such that the piston 55 is released to move forward to its advanced second position, driven by the spring 61.

As shown in fig. 4, in the first position of the piston 55 an air chamber 58 in the cylinder 60 has a volume of air of, by way of example, about 820 mm³, between the head 54 of the piston 55 and a dispensing structure generally indicated by numeral 90.

As the piston 55 is displaced by the user towards the retracted first position by rotation of the rotatable head 28, air is simultaneously drawn into this air chamber 58 through an air access port. The discharge opening 25 for discharging the mist may be used for this purpose; however, to reduce the risk of contamination of the air chamber 58 by already dispensed liquid fluid being drawn back in together with fresh air the air access port is preferably located elsewhere, such as in the peripheral wall of the cylinder 60 and is preferably equipped with a one-way valve (not shown). Another not shown one-way valve may be located in connection with the discharge opening 25 to prevent or restrict air entry, to prevent the aforementioned contamination. In this manner there is a reduced need for using liquid fluids with preservatives.

The dispensing structure 90 is preferably inserted into the cylinder 60 to define the foremost end thereof, opposite the rear closure 62. As shown, a passage 91 for a first air flow leads from the air chamber 58 into an internal liquid chamber 92 within the structure 90. One or more secondary passages 97, such as three, preferably winding (eg. spirally) around the dispensing structure 90 as in the shown embodiment, are formed and arranged to communicate with the air chamber 58 on the one hand and with a mixing chamber 95 on the other hand. The mixing chamber 95 is located in front of the dispensing structure 90 and defined between the dispensing structure 90 and a front casing part 23 that has an aperture defining the discharge opening 25. The one or more secondary passages 97, which may have circular cross-section(s), allow for a second air flow to flow past the structure 90 and, hence, the liquid chamber 92, i.e. not through the liquid chamber 92, to enter the mixing chamber 95.

The dispensing structure 90 may be provided with a laterally oriented port or passage to allow for a liquid flow to the internal liquid fluid chamber 92 from the second portion 81' of the tube 80 via connection point 81", and also has a discharge opening 93 opposite the passage 91. The mixing chamber 95 may, as shown in figs. 4 and 5, follow the general contour of a saucer, with opening 25 being opposite and aligned with the more narrow opening 93 and with the secondary passages 97 opening into the mixing chamber 95 at the "rim" of the saucer. Valves (not shown) opening at a certain pressure may be provided at the normally narrow discharge opening 93 and in passage 91 to retain liquid fluid in the liquid fluid chamber 92, but may be dispensed with, in particular where the liquid fluid has a relatively high viscosity.

The volume of the liquid fluid chamber 92 corresponds to the aforementioned volume of the dose of liquid fluid dispensed or dosed by the peristaltic pump 82 of the prior device 1 upon correct manipulation thereof, i.e. proper rotation of the disc 84 through a given angle, by means of handle 26, and may by way of example be in the order of about 50 mm³. The passage 91, opening 93 and fluid chamber 92 may have a circular cross-section.

When preparing the prior device 1 for use after a previous discharge/dispensing or when taking the device 1 into use for the first time, a person will fill the fluid chamber 92, in the prior device 1 using the peristaltic pump 82, and also withdraw the piston 55 to the retracted, first position. This may, in principle, occur in any sequence, or simultaneously if a single operating part is used for the peristaltic pump 82 and the piston 55. In fig. 4 the fluid in fluid chamber 92 is represented by dots.

On subsequent release of the first drive, in the shown embodiment constituted by the spring 61 which advances the piston 55, air in the air chamber 58 drawn into the cylinder 60 during the preceding withdrawal of the piston 55 is forced out of the cylinder 60 into the liquid fluid chamber 92 within the structure 90 through the passage 91, and preferably also leaves the cylinder 60 as the aforementioned second air flow through the series of additional passages 97 opening up into the mixing chamber 95. The terms "first" and "second" used in this respect are not intended to imply any necessary time wise delay between the two flows of air but primarily to reflect that several flows of air are generated, normally having different purposes, the first air flow primarily serving to force liquid fluid out of the liquid fluid chamber 92. After all liquid fluid has been forced/driven out of liquid fluid chamber 92 any remaining air in the air chamber 58 may still be discharged into the mixing chamber 95 through the liquid fluid chamber 92, to flush the fluid chamber 92.

Turning now to fig. 6, this drawing shows a perspective partial and cross-sectional view of the front end F of an embodiment of the device 1 of the presently claimed invention, illustrating a part of the casing 20, the eyelid opener portion 10, a bottom structure B at front or bottom end F of the casing 20 including a fluid chamber 92 for holding a first volume or dose of the liquid to be dispensed, the discharge opening 25 and the mixing chamber 95, as well as an air chamber 58 communicating in this embodiment - through conduits 91, 97 for a first flow of air and another flow of air, respectively, formed in the bottom structure B - with the mixing chamber 95 as well as with the fluid chamber 92 so as to deliver separate flows of air from the air chamber 58 in the same manner discussed above with reference to the device of fig. 1, and as defined in the appended claim 2. The bottom structure B is conveniently configured for easy assembly with the casing 20, such as by snap-action.

In other embodiments, as defined broadly in claim 1, the air chamber 58 may be configured to communicate only with the mixing chamber 95, i.e. not directly with the fluid chamber 92, so that liquid is drawn into the mixing chamber 95 by air through a venturi effect only, or to communicate only with the fluid chamber 92 so that liquid is driven into the mixing chamber 95 solely by the effect of the air flowing into the fluid chamber 92, purging all liquid therein.

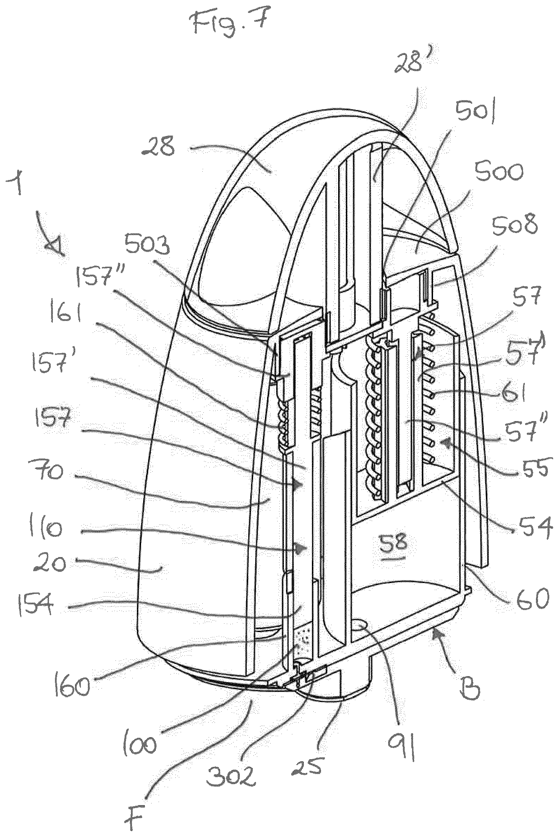

Fig. 7 shows another perspective partial and cross-sectional view of the device 1 of fig. 6, without the eyelid opener structure. Shown to the right in the drawing is a first drive that comprises a spring 61, shown as a mechanical spring, an air piston 55 including a piston rod assembly with a piston rod 57" and a piston head 54 displaceably received inside a cylindrical structure 60 defining the aforementioned air chamber 58, the spring 61 acting on the piston rod 57".

The rotatable head 28 of the device 1 has a tubular extension 28' and is configured for snap-engagement with the casing in the area of a shelf 500 which has a central aperture for receiving the tubular extension 28' and which has downwardly protruding sockets 501, 508 for receiving the upper part of various piston rod assemblies discussed further below. Projecting below the shelf 500 the tubular extension 28' is provided with a peripheral toothing (not shown) engaged with respective toothing of a first drive operable to draw in air into the air chamber 58 and to expel that air and a second drive operable to supply a second volume, corresponding to two or possibly more doses, of the liquid from a supply to a dosing chamber having a corresponding capacity and for emptying the dosing chamber by repetitive delivery of single doses towards the fluid chamber 92.

On energizing or activating the first drive, such as by rotating the head 28, the air piston head 54 is first displaced to a most retracted position, shown in fig. 7, away from the bottom structure B while compressing the spring 61. The first drive, the operation of which will be described later, further comprises, or is supplemented by, a hold and release mechanism 30 for holding the air piston 55 in two different positions until release such that the spring 61 operates to drive the air piston head 54 forwards in two steps, by the user activating the release mechanism each time, each forward movement of the air piston head 54 from a retracted position resulting in a corresponding expelling of an amount of the air inside the air chamber 58. Referring to fig. 7, one such position is shown therein, while another such position would be at about halfway down towards the bottom structure B that defines the end of the air chamber 58.

Shown to the left in fig. 7 is also a second drive that comprises a spring 161, shown as a mechanical spring, a piston for driving a liquid, referred to in the following as "liquid piston" 110 including a rod 157 and a piston head 154 displaceably received inside a piston cylinder in the form of cylindrical structure 160 defining a dosing chamber 100, the spring 161 acting on the piston rod 157' of the piston rod assembly.

On energizing or activating the second drive, such as by rotating the head 28 and simultaneously energizing or activating the first drive in the manner described above, engagement with the toothing on the tubular extension 28' of the head 28 brings about a rotational movement of the liquid piston 155, displacing it, at the same time, first to a most retracted position away from the bottom structure B while compressing the spring 161. The second drive, the operation of which will also be described later, further comprises, or is supplemented by, a hold and release mechanism 130 for holding the liquid piston 155 in one of two different positions until release, the spring 161 operating to drive the liquid piston 155 forwards in two steps, timed by the user activating the release mechanism, each forward movement towards the structure B of the liquid piston 10 from the most retracted position resulting in a dose being delivered to fluid chamber 92, as explained below.

Figs. 8a and 8b show the parts of an embodiment of the bottom structure B, with a first part 200 including on the lower side thereof (not visible in fig. 8b) the nozzle 25, and configured to be assembled with a second part 210, the first part 200 having two recesses 201, 202 formed in a surface S thereof, the recesses 201, 202 defining two respective conduits 301, 302 when the first part 200 is assembled with the second part 210. The conduits 302, 301, respectively, lead from the supply 70 to the dosing chamber 100 and from the dosing chamber 100 to the fluid chamber 92 and preferably each includes a one-way valve 321, 322, such as elastic flaps, configured for blocking any fluid flow towards said supply 70 on the liquid being supplied to the dosing chamber 100. The first part 200 preferably includes the passage 93 connecting the liquid chamber 92 with the mixing chamber 95 and/or a passage 91 connecting the air chamber 58 with the liquid chamber 92 and/or one or more passages 97 separately connecting the air chamber 58 with the mixing chamber 95. The one or more separate passages 97 may be arranged winding around the fluid chamber 92, as discussed in connection with figs. 1-5. As shown, it is preferred to mould the piston cylinders 60, 161 integrally with the second part 210.

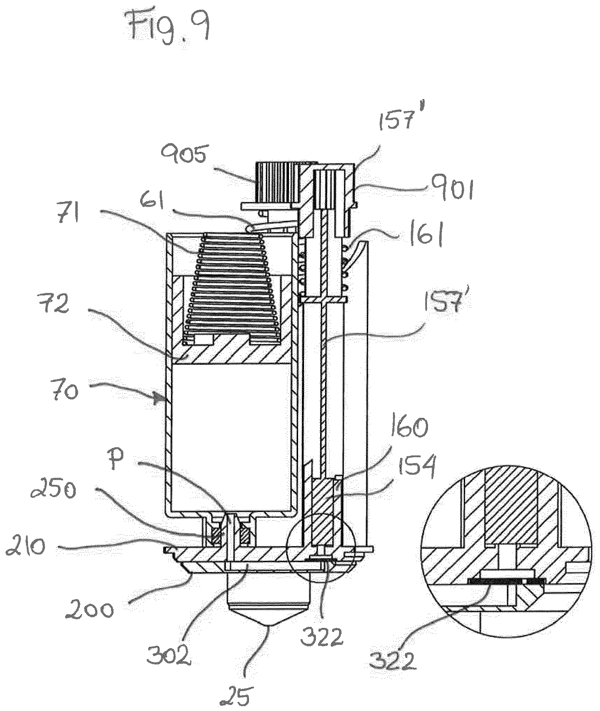

Fig. 9 is a cross-sectional view, showing the liquid piston 154 in its most advanced position and with a supply in the form of a container 70 attached to a spigot-like connector 250 integral with the second part 210 of the bottom structure B and configured with a passage P communicating with the aforementioned conduit 302 leading to the dosing chamber 100. In this embodiment, the container 70 has at one end a compressed spring 71 acting on a displaceable bottom 71 of the container 70 and a rubber membrane at the other end, the membrane being configured to be penetrated by the spigot 250 to establish a fluid connection. It will be understood that, as the piston head 154 is raised to a retracted position liquid is drawn into the dosing chamber 100 through conduit 302, with one-way valve 322 being open and valve 321 being closed. After filling of the dosing chamber 100, i.e. when the piston head 154 is in the position shown in fig. 7 such that the dosing chamber 100 contains two doses of the liquid (illustrated by the dots in fig. 7), and when discharge of one dose from the dosing chamber 100 to the fluid chamber 92 is desired, valve 322 closes automatically by the pressure increase in the dosing chamber 100 as the piston head 154 moves downward, and valve 321 opens. Some priming typically may be required, depending on the length on the conduit 301, as obviously the liquid discharged from the dosing chamber 100 pushes forward any liquid already standing in the conduit 301 leading from the dosing chamber 100 to the fluid chamber 92.

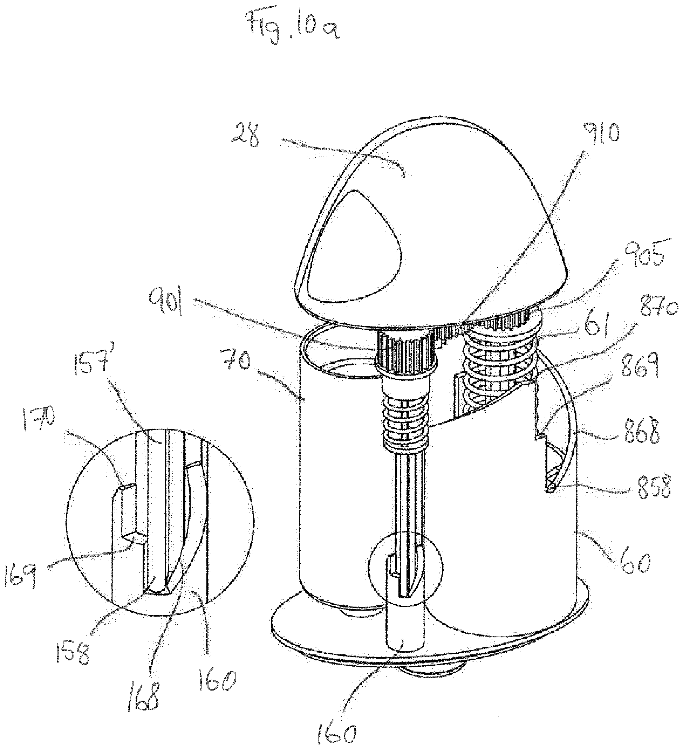

Figs. 10a-10e illustrate the movements of the various components of the device 1 as the handle 28 and, hence, the first and second drives including the hold and release mechanisms, are operated. For simplicity, certain structural components, including shelf 500, are not shown.

Operation of the device 1 starts with both piston heads 54, 154 is the most advanced, forward position close to the bottom structure B, for the liquid piston as shown in fig. 9. Rotation of the teeth or gear 910 on tubular extension 28' brings about a rotation, determined by the chosen gearing, of teeth/gear 901, 905 that are integral with upper parts 157" and 57", respectively, of the piston rod assemblies 157, 57 of the liquid piston 110 and air piston 55, respectively. A lower part 157', 57' of each piston rod assembly carries the respective piston head 154, 54 and is connected to the corresponding upper part 157", 57" so as to rotate therewith while at the same time being axially displaced relative to the upper part, under the action of the corresponding spring 61, 161 on the one hand and a winding cam surface 168, 868 formed on the respective cylindrical structure 60, 160 that defines the air chamber 58 and the liquid chamber 100, through a respective projection 158, 858 on the lower parts 157", 57' that rides on the corresponding cam surface 168, 868.

During this displacement, the respective piston heads 54, 154 move away from the bottom structure B, drawing liquid into the liquid chamber 100 from the fluid supply 70 through conduit 302 and air into the air chamber 54 through any suitable passage formed in the cylindrical wall 60, wherein such suitable passage may preferably be provided with a one-way valve.

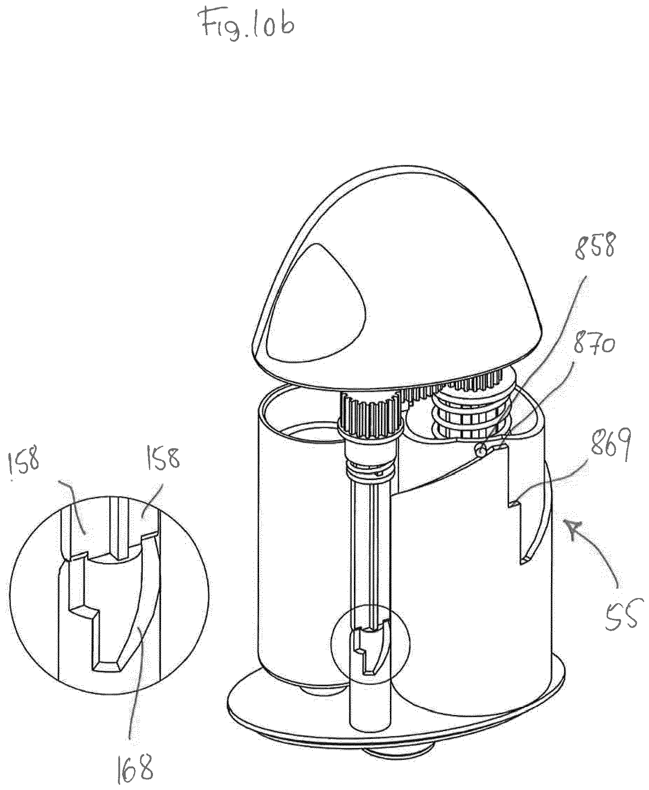

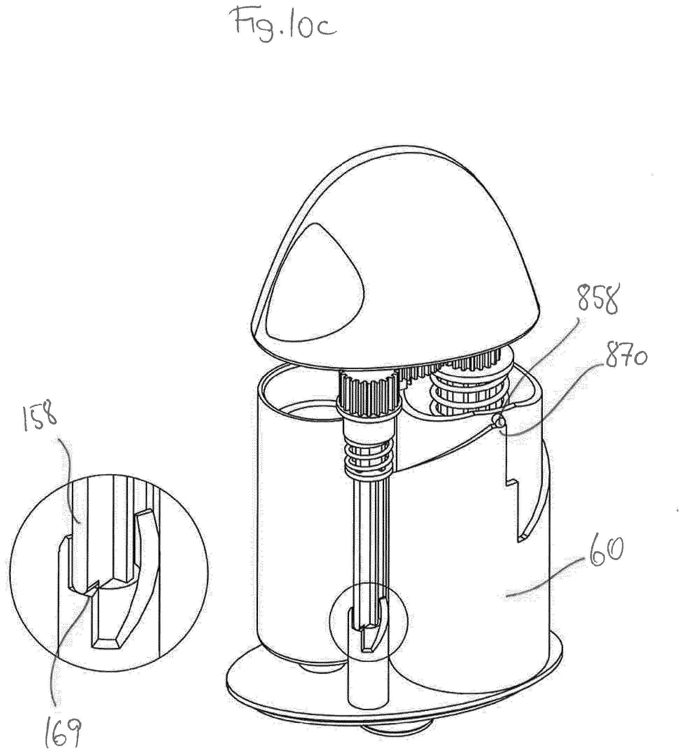

The cam surfaces 168, 868 are each followed by a stepped control surface which includes two steps 169 and 170, and two steps 869 and 870, respectively. The stepped control surfaces are so adapted that when the handle 28 has been fully rotated, such as by 180°, and stopped by a stop device (not shown), past the near-end position of the projections 158, 858 shown in fig. 10b, the projection 858 of the air piston 55 will rest on the upper step 870 as shown in fig. 10c while at the same time the projection 158 of the liquid piston has been rotated past the upper step 170 whereby the lower part 157' of the piston rod assembly 157, together with liquid piston head 154 is driven forward towards the base structure B by the spring 161 until the projection 158 reaches the second step 169. At this moment, air chamber 54 is filled to its maximum capacity with air while half of the liquid that was previously drawn into the dosing chamber 100 at the point shown in fig. 10b has been delivered to the fluid chamber 92 through the downward movement of the liquid piston head 154 to the position defined by the second, lower step 169. By appropriately selecting the difference in height between two steps 169, 170 it may be ensured that exactly half the amount of liquid contained in the dosing chamber 100 is delivered towards the fluid chamber 92, and this amount or volume preferably is set to correspond to one dose of the liquid fluid to be dispensed. Any liquid contained in conduit 301 will be purged, but the dose delivered to the fluid chamber 92 will nevertheless correspond to one dose of the liquid, part of which liquid may have been residing on the conduit 302 since the last time a dispensing was carried out.

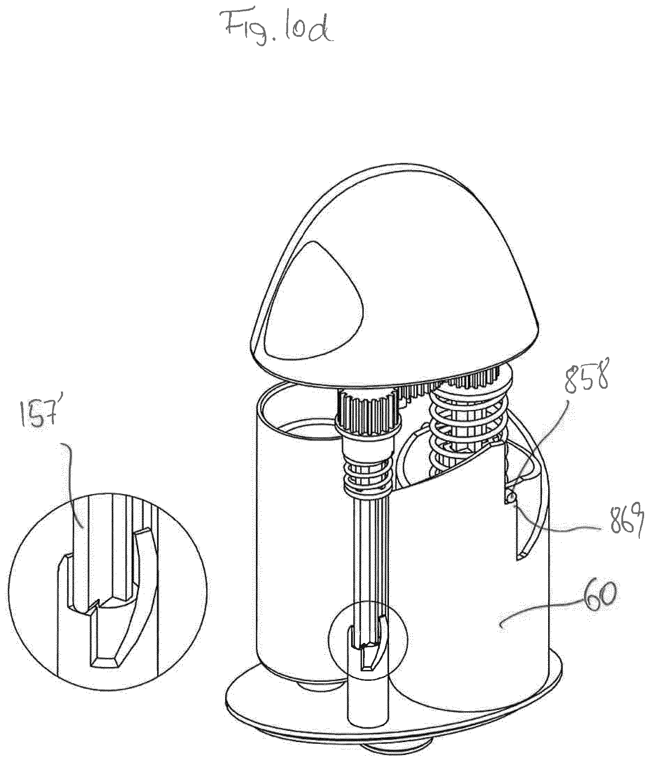

The device 1 in the condition shown in fig. 10c is now ready for dispensing a mist of the liquid contained in the fluid chamber 92, and the user now holds the device 1 against his eye. At this point, activation or manipulation of a hold-and-release mechanism is carried out, the purpose of which is to drive the projection 858 out of engagement with the step 870 of the air cylinder casing 60, by a slight rotation of the relevant part 57' of the air piston assembly such that the projection 858 is forced by spring 61 to rest on the second step 869 on the air cylinder casing 60, as shown in fig. 10d, the air piston 54 being thereby advanced one step inside the air cylinder at the same time discharging half the air inside, through passages 91 and/or 97, driving at the same time liquid out from the fluid chamber 92 and generating a mist of the liquid through the interaction between the air and the liquid.

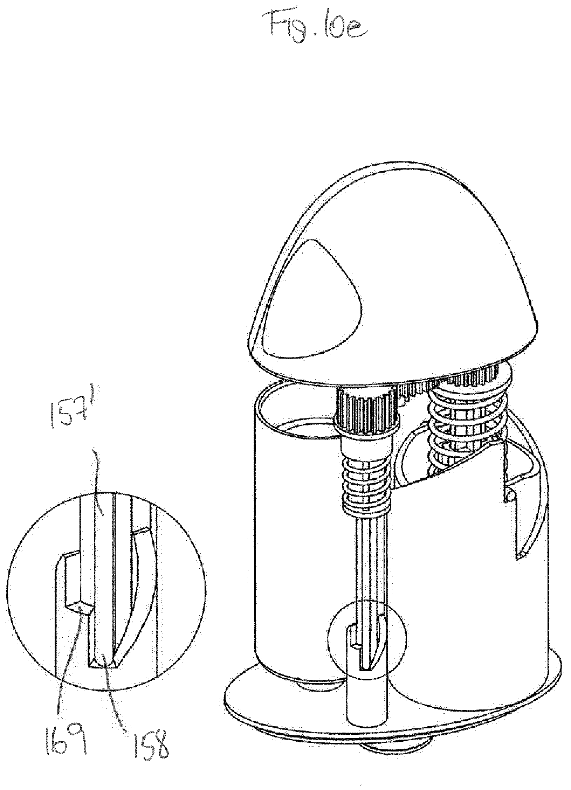

The user will now want to apply the ophthalmic fluid to the other eye, for which he manipulates once again the hold-and-release mechanism to rotate now the lower part 157' of the liquid piston rod assembly such that projection 158 thereof moves past the second step 169 of the dosing chamber casing 160 to its original position, at the same time driving the remaining dose in the dosing chamber 100 into the empty fluid chamber 92, cf. fig. 10e. A last manipulation of the hold-and-release mechanism drives the projection 858 out of engagement with the step 869 of the air cylinder casing 60, by a slight rotation of the relevant part 57' of the air piston assembly such that the projection 858 is brought to rest on the lowermost step 858 on the air cylinder casing 60 while the air piston 54 is advanced one last step inside the air cylinder at the same time discharging the remaining air inside, through passages 91 and/or 97, driving at the same time liquid out from the fluid chamber 92 and generating a mist of the liquid through the interaction between the air and the liquid.

Rotation of the piston assemblies to bring the projections158, 868 out of engagement with the corresponding steps may be by pressing several times a tab 30 as shown in fig. 1, effecting each time a rotation of the piston heads through a cam face (now shown), or by further rotation of the head 28 in small increments, limited by stops. As such, the hold-and-release mechanisms 30, 130 referred to above may be formed by a single mechanism. By way of example, a coupling structure may be incorporated such that liquid piston and, hence, the projection 157', rotates automatically (by being directly or indirectly coupled to the air piston) when the air piston projection 858 has reached the position shown in fig. 10d, thereby moving to the most forward position shown in fig. 10e. A mechanism (not shown) allowing the liquid piston to rotate without the handle 28 rotating at the same time may be included, i.e. by adapting the gearing correspondingly.

It will be understood that the speed of the second flow of air leaving the air chamber 58 is preferably high and that the first flow of air discharged by the action of the spring 61 ejects or pushes the liquid fluid out from the liquid fluid chamber 92 through the discharge opening 93 at the forward end of the liquid fluid chamber 92, into the mixing chamber 95. Flow of the liquid fluid into the mixing chamber 95 may be assisted by any venturi effect arising from the inflow into the mixing chamber 95 of the secondary air flow. A fine mist of the liquid fluid is generated, even where the fluid has a medium to high viscosity, by the second air flow leaving the secondary passages 97 impinging on the simultaneously ejected liquid fluid, in front of the discharge opening 93.

It is contemplated to design the course of the one or more passages 97 such that the forward second air flow preferably strikes the ejected liquid fluid at an acute angle with respect to the general forward direction of flow of the ejected liquid fluid. The fine mist generated thereby exits the device 1 through the discharge opening 25 with high inertia, as determined i.a. by the spring 61, towards the eye of the user, which eye is held open by the eyelid opener portion 10. By properly adjusting the volume of air in the air chamber 58 it may be ensured that all dispensed fluid is blown away from the device 1 during each dispensing.

By selecting the relative dimensions of the one or more passages 97 in relation to the dimension of passage 91 leading into the liquid fluid chamber 92 and the discharge opening 93 leading out there from any desired flow and droplet size may be obtained. It may in some instances be preferred that the liquid fluid is ejected into a second flow of air already prevailing in the mixing chamber 95. The design of the discharge opening 25, normally located about 20-30 mm from the eye when the device 1 is correctly held, ensures that the mist is deposited only on the eye, and this may be achieved with the device 1 held in any orientation, horizontally or vertically.

The container 70 may, in one embodiment, constitute a collapsible bag or pouch made eg. from thin and/or flexible foil; possibly closed by welding. Alternatively, the container 70 may constitute a moulded plastic component. The container 70 may, in one embodiment, constitute a cylinder including a movable piston. The movable piston may define the bottom of the cylinder and the distal fixed end may be provided with means for hydraulic and/or mechanical interface to the device 1. Means, such as a spring 71, may be provided in order to urge or push the piston into the cylinder. By this, liquid dosing may be facilitated.

The container 70 may, in one embodiment, be joined to a preferably moulded plastic component allowing for hydraulic and/or mechanical interface to the device 1.

The liquid container 70 may be integrated with the wet part of the device 1, preferably in a manner allowing for exchangeability of the container 70. The separate container 70 may have a unique shape for mechanical orientation and/or fixation of the container with respect to the device 1.

All components of the devices mentioned above may be of any desired material, such as a plastics material, a metal material or combinations thereof. Notably, the collar for keeping the user's eyelid open may carry a surface material with antibacterial properties or be formed from such a material. Furthermore, the components and/or surfaces defining and/or surrounding the discharge opening 25 and/or nozzle may be made from a material with antibacterial properties. Additionally, the material defining the inside surface of the fluid chamber 95 may have hydrophobic properties to assist in discharging all liquid held therein.

As can be seen the device 1 of the shown embodiment of the present invention has a relatively flat casing 20, such as one with a length and width in the order of about 90mm and 60 mm, respectively, allowing it to be conveniently held by the user.

Additionally, when the supply container 70 is empty or nearly empty and safe delivery of a dose can no longer be performed an electronical or mechanical system (not shown) may be provided either to monitor the number of activations of the handle 28 or the remaining content of liquid in the container 70. The mechanical system may be configured to block the movement of the handle. In one embodiment, an element connected to and driven by the handle 28 is fitted with a protruding tab, and the element increments its position at each operation of the handle 28; when the accumulated number of increments corresponds to the available number of doses the protruding tab stops against a stop to block further movement of the handle 28 until reset, such as when a new container 70 is inserted into the device 1.

While it may be preferred to configure the device according to the invention such that first and second flows of air are established, which may be preferred where the liquid fluid has a high viscosity, in other cases it may not be required to provide for a secondary flow of air, by configuring the discharge opening 25 as a nozzle suitable for establishing by its geometry alone a small droplet size of the liquid fluid dispensed by the first drive, such as by incorporating a fine mesh structure. For the purpose of the present invention, dispensing of doses preselected in the range of 6µl - 40µl is considered highly relevant.

## Claims

1. A device (1) for dispensing a mist of a dose of an ophthalmic liquid fluid, comprising:
- a discharge opening (25) for said mist,
- a mixing chamber (95) being in fluid flow communication with said discharge opening (25),
- an air chamber (58),
- a first drive operable to expel air from said air chamber (58),
- a fluid chamber (92) for holding a first volume of said liquid and being in fluid flow communication with said mixing chamber (95),
- a dosing chamber (100) for containing a second volume of said liquid corresponding to a plurality of said doses, preferably to two doses, of said liquid,
- said dosing chamber (100) being in fluid flow communication with or configured for being in fluid flow communication with said fluid chamber (92), **characterised by**
said dosing chamber (100) also being in fluid flow communication with or configured for being in fluid flow communication with a supply (70) of said liquid, such as a collapsible liquid container or a liquid container having a movable bottom,
- a second drive operable to supply said second volume of said liquid from said supply (70) to said dosing chamber (100) and for emptying said dosing chamber (100) by repetitive delivery of single doses of said plurality of doses of said liquid contained therein towards said fluid chamber (92),
- said air chamber (58) being in fluid flow communication with said fluid chamber (92) for said expelled air to drive said liquid delivered to said fluid chamber (92) into said mixing chamber (95) by said expelled air.

2. The device (1) according to claim 1,
- said air chamber (58) being in fluid flow communication with said fluid chamber (92) and separately with said mixing chamber (95),
- said device (1) being configured for establishing one flow of said expelled air flowing into said fluid chamber (92), so as to force said fluid insaid fluid chamber (92) into said mixing chamber (95), and another flow of said expelled air flowing past said fluid chamber (92) into said mixing chamber (95), for assisted atomizing of said liquid by said other flow of air striking said liquid forced into said mixing chamber (95).

3. The device (1) according to claim 1 or 2, said air chamber (58) being configured to contain a volume of air sufficient for two consecutive ones of said expellings of air, each consecutive expelling driving a respective single dose contained in said fluid chamber (92) into said mixing chamber (95).

4. The device (1) according to any of the previous claims, said fluid chamber (92) being configured with a volume corresponding to the volume of said dose of said liquid.

5. The device (1) according to any of the previous claims, comprising an energizer, such as a pressurised canister or a rotatable handle (28), for energizing, preferably in one operation, said first drive and said second drive, wherein energizing of said first drive and of said second drive provides energy required for at least two consecutive ones of said expellings of air from said air chamber (58) and at least two consecutive ones of said delivery of one dose to said fluid chamber (92), respectively.

6. The device (1) according to any of the previous claims, said first drive comprising:
- a spring (61), such as a mechanical spring,
- an air piston (55) including a piston head (54) displaceably received inside a structure (60), such as a cylinder, defining said air chamber (58),
- said spring (61) acting on said air piston (55),
- said energizing, or activation of said first drive, displacing said air piston (55) to a retracted position while compressing said spring (61),
- the device (1) further comprising a hold and release mechanism (30) for holding and releasing said air piston (55) in and from said retracted position, for said spring (61) to drive said air piston (55) towards an advanced position and providing for said expelling.

7. The device according to the previous claim, said hold and release mechanism (30) being configured to provide a stepwise advancing of said air piston (55) to multiple advanced positions.

8. The device according to any of the previous claims, said second drive comprising:
- a spring (161), such as a mechanical spring,
- a liquid piston (110) including a piston head (154) displacably received inside said dosing chamber (100),
- said spring (161) of said second drive acting on said liquid piston (110),
- said energizing, or activation of said second drive, displacing said liquid piston (110) to a retracted position while compressing said spring (161) and drawing said liquid into said dosing chamber (100) from said supply (70),
- the device (1) further comprising a hold and release mechanism (130) for holding and releasing said liquid piston (110) in and from said retracted position, for said spring (161) of said second drive to drive said liquid piston (110) towards an advanced position for said delivery of a single dose of said fluid to said fluid chamber (92).

9. The device according to the previous claim, said hold and release mechanism (130) being configured to provide a stepwise advancing of said liquid piston (110) to multiple advanced positions.

10. The device according to any of the previous claims, comprising a structure (B) with a first part (200), preferably including said nozzle (25), and connected with a second part (210), said first part (200) or and/or said second part (210) having one or more recesses (201, 202) formed in a surface (S) thereof, said recesses (201, 202) defining at least one conduit (301, 302) when said first part (200) is assembled with said second part (210), said conduit (301, 302) leading from said supply (70) to said dosing chamber (100) and/or from said dosing chamber (100) to said fluid chamber (92), said conduit (301, 302) optionally including one-way valves (321, 322), such as elastic flaps, configured for blocking any fluid flow towards said supply (70) on said liquid being supplied to said dosing chamber (100), said first part (210) preferably including a passage (93) connecting said liquid chamber (92) with said mixing chamber (95) and/or a passage (91) connecting said air chamber (58) with said liquid chamber (92) and/or one or more passages (97) separately connecting said air chamber (58) with said mixing chamber (95), said one or more separate passages (97) optionally winding around said fluid chamber (92).

## Patentansprüche

1. Vorrichtung (1) zur Abgabe eines Nebels einer Dosis eines flüssigen Augenfluids, umfassend:
- eine Auslassöffnung (25) für den Nebel,
- eine Mischkammer (95), die mit der Auslassöffnung (25) in Fluidströmungsverbindung ist,
- eine Luftkammer (58),
- einen ersten Antrieb, der zum Ausstoßen von Luft von der Luftkammer (58) betrieben werden kann,
- eine Fluidkammer (92) zum Aufnehmen eines ersten Volumens der Flüssigkeit und die mit der Mischkammer (95) in Fluidströmungsverbindung ist,
- eine Dosierungskammer (100) zum Enthalten eines zweiten Volumens der Flüssigkeit entsprechend einer Mehrheit von Dosen, vorzugsweise zwei Dosen, der Flüssigkeit,
- wobei die Dosierungskammer (100) mit der Fluidkammer (92) in Fluidströmungsverbindung ist oder dafür eingerichtet ist, **dadurch gekennzeichnet, dass** die Dosierungskammer (100) auch mit einer Versorgung (70) der Flüssigkeit in Fluidströmungsverbindung ist oder dafür eingerichtet ist, wie beispielsweise ein zusammendrückbarer Flüssigkeitsbehälter oder ein Flüssigkeitsbehälter mit einem beweglichen Boden,
- einen zweiten Antrieb, der zum Zuführen des zweiten Volumens der Flüssigkeit von der Versorgung (70) zur Dosierungskammer (100) und zum Entleeren der Dosierungskammer (100) durch wiederholte Abgabe von Einzeldosen der Mehrheit von Dosen der darin enthaltenen Flüssigkeit in Richtung der Fluidkammer (92) betrieben werden kann,
- wobei die Luftkammer (58) mit der Fluidkammer (92) in Fluidströmungsverbindung ist, damit die ausgestoßene Luft, die Flüssigkeit, die an die Fluidkammer (92) abgegeben wird, durch die ausgestoßene Luft in die Mischkammer (95) treibt.

2. Vorrichtung (1) nach Anspruch 1,
- wobei die Luftkammer (58) mit der Fluidkammer (92) in Fluidströmungsverbindung ist und mit der Mischkammer (95) getrennt ist,
- wobei die Vorrichtung (1) dafür ausgelegt ist, eine Strömung von ausgestoßener Luft, die in die Fluidkammer (92) strömt, herzustellen, um das Fluid in der Fluidkammer (92) in die Mischkammer (95) zu treiben, und wobei eine andere Strömung der ausgestoßenen Luft der Fluidkammer (92) vorbei und in die Mischkammer (95) strömt zur unterstützten Zerstäubung der Flüssigkeit durch die andere Strömung von Luft, die die in die Mischkammer (95) getriebene Flüssigkeit trifft.

3. Vorrichtung (1) nach Anspruch 1 oder 2, wobei die Luftkammer (58) dafür eingerichtet ist, ein Volumen von Luft zu enthalten, ausreichend für zwei aufeinanderfolgende der Ausstöße von Luft, wobei jeder aufeinanderfolgende Ausstoß eine jeweilige in der Fluidkammer (92) enthaltene Einzeldosis in die Mischkammer (95) treibt.

4. Vorrichtung (1) nach einem der vorgehenden Ansprüche, wobei die Fluidkammer (92) mit einem Volumen entsprechend dem Volumen der Dosis der Flüssigkeit ausgelegt ist.

5. Vorrichtung (1) nach einem der vorgehenden Ansprüche, umfassend einen Energiespender, wie einen unter Druck stehenden Behälter oder einen drehbaren Griff (28), zum Versorgen, vorzugsweise in einem Vorgang, des ersten Antriebs und des zweiten Antriebs mit Energie, wobei die Versorgung des ersten Antriebs und des zweiten Antriebs mit Energie Energie bereitstellt, die für mindestens zwei aufeinanderfolgende der Ausstöße von Luft von der Luftkammer (58) bzw. mindestens zwei aufeinanderfolgende der Abgabe einer Dosis an die Fluidkammer (92) erforderlich ist.

6. Vorrichtung (1) nach einem der vorgehenden Ansprüche, wobei der erste Antrieb Folgendes umfasst:
- eine Feder (61) wie eine mechanische Feder,
- einen Luftkolben (55) mit einem Kolbenkopf (54), die in einer Struktur (60), wie einem Zylinder, die die Luftkammer (58) definiert, verschieblich aufgenommen ist,
- wobei die Feder (61) auf den Luftkolben (55) einwirkt,
- wobei die Versorgung mit Energie, oder die Aktivierung des ersten Antriebs, den Luftkolben (55) in eine zurückgezogene Position verschiebt, während die Feder (61) zusammengedrückt wird,
- wobei die Vorrichtung (1) weiter einen Halte- und Freigabemechanismus (30) zum Halten und Freigeben des Luftkolbens (55) in und von der zurückgezogenen Position umfasst, damit die Feder (61) den Luftkolben (55) gegen eine vorgerückte Position treibt und das Ausstoßen vorsieht.

7. Vorrichtung nach dem vorgehenden Anspruch, wobei der Halte- und Freigabemechanismus (30) dafür eingerichtet ist, eine schrittweise Vorführung des Luftkolbens (55) in mehrere vorgerückte Positionen vorzusehen.

8. Vorrichtung nach einem der vorgehenden Ansprüche, wobei der zweite Antrieb Folgendes umfasst:
- eine Feder (161) wie eine mechanische Feder,
- einen Flüssigkeitskolben (110) mit einem Kolbenkopf (154), der in der Dosierungskammer (100) verschieblich aufgenommen ist,
- wobei die Feder (161) des zweiten Antriebs auf den Flüssigkeitskolben (110) einwirkt,
- wobei die Versorgung mit Energie, oder die Aktivierung des zweiten Antriebs, den Flüssigkeitskolben (110) in eine zurückgezogene Position verschiebt, während die Feder (161) zusammengedrückt wird und die Flüssigkeit in die Dosierungskammer (100) von der Versorgung (70) gezogen wird,
- wobei die Vorrichtung (1) weiter einen Halte- und Freigabemechanismus (130) zum Halten und Freigeben des Flüssigkeitskolbens (110) in und von der zurückgezogenen Position umfasst, damit die Feder (161) des zweiten Antriebs den Flüssigkeitskolben (110) gegen eine vorgerückte Position für die Abgabe einer Einzeldosis des Fluids zur Fluidkammer (92) treibt.

9. Vorrichtung nach dem vorgehenden Anspruch, wobei der Halte- und Freigabemechanismus (130) dafür eingerichtet ist, eine schrittweise Vorführung des Flüssigkeitskolbens (110) in mehrere vorgerückte Positionen vorzusehen.

10. Vorrichtung nach einem der vorgehenden Ansprüche, umfassend eine Struktur (B) mit einem ersten Teil (200), vorzugsweise einschließlich der Düse (25), und verbunden mit einem zweiten Teil (210), wobei der erste Teil (200) und/oder der zweite Teil (210) eine oder mehrere Ausnehmungen (201, 202), gebildet in einer Oberfläche (S) davon, aufweist bzw. aufweisen, wobei die Ausnehmungen (201, 202) mindestens eine Leitung (301, 302) definieren, wenn der erste Teil (200) mit dem zweiten Teil (210) zusammengefügt ist, wobei die Leitung (301, 302) von der Versorgung (70) in die Dosierungskammer (100) und/oder von der Dosierungskammer (100) in die Fluidkammer (92) führt, wobei die Leitung (301, 302) eventuell Einwegventile (321, 322), wie elastische Laschen, umfasst, ausgelegt zum Blockieren einer Fluidströmung gegen die Versorgung (70) auf der Flüssigkeit, die zur Dosierungskammer (100) zugeführt wird, wobei der erste Teil (210) vorzugsweise einen Durchgang (93), der die Flüssigkeitskammer (92) mit der Mischkammer(95) verbindet, und/oder einen Durchgang (91), der die Luftkammer (58) mit der Flüssigkeitskammer (92) verbindet und/oder einen oder mehrere Durchgänge (97), die getrennt die Luftkammer (58) mit der Mischkammer (95) verbindet, umfasst, wobei der eine oder mehrere getrennte Durchgänge (97) eventuell um die Fluidkammer (92) umwickelt ist.

## Revendications

1. Dispositif (1) pour distribuer un brouillard d'une dose d'un fluide liquide ophtalmique, comprenant :
- une ouverture d'évacuation (25) pour ledit brouillard,
- une chambre de mélange (95) étant en communication d'écoulement de fluide avec ladite ouverture d'évacuation (25),
- une chambre à air (58),
- un premier entraînement pouvant expulser l'air de ladite chambre à air (58),
- une chambre à fluide (92) pour maintenir un premier volume dudit liquide et étant en communication d'écoulement de fluide avec ladite chambre de mélange (95),
- une chambre de dosage (100) pour contenir un deuxième volume dudit liquide correspondant à une pluralité desdites doses, de préférence à deux doses, dudit liquide,
- ladite chambre de dosage (100) étant en communication d'écoulement de fluide avec ou configurée pour être en communication d'écoulement de fluide avec ladite chambre à fluide (92), **caractérisé en ce que** ladite chambre de dosage (100) est aussi en communication d'écoulement de fluide avec ou configurée pour être en communication d'écoulement de fluide avec une alimentation (70) dudit liquide, telle qu'un récipient de liquide pliable ou un récipient de liquide à fond mobile,
- un deuxième entraînement pouvant fonctionner pour fournir ledit deuxième volume dudit liquide provenant de ladite alimentation (70) à ladite chambre de dosage (100) et pour vider ladite chambre de dosage (100) par administration répétitive de doses uniques de ladite pluralité de doses dudit liquide contenu à l'intérieur de celle-ci vers ladite chambre à fluide (92),
- ladite chambre à air (58) étant en communication d'écoulement de fluide avec ladite chambre à fluide (92) pour que ledit air expulsé entraîne ledit liquide délivré à ladite chambre à fluide (92) dans ladite chambre de mélange (95) par ledit l'air expulsé.

2. Dispositif (1) selon la revendication 1,
- ladite chambre à air (58) étant en communication d'écoulement de fluide avec ladite chambre à fluide (92) et séparément avec ladite chambre de mélange (95),
- ledit dispositif (1) étant configuré pour établir un écoulement dudit air expulsé s'écoulant dans ladite chambre à fluide (92), pour forcer ledit fluide de ladite chambre à fluide (92) dans ladite chambre de mélange (95), et un autre écoulement dudit air expulsé s'écoulant devant ladite chambre à fluide (92) dans ladite chambre de mélange (95), pour une pulvérisation assistée dudit liquide par ledit autre flux d'air heurtant ledit liquide forcé dans ladite chambre de mélange (95).

3. Dispositif (1) selon la revendication 1 ou 2, ladite chambre à air (58) étant configurée pour contenir un volume d'air suffisant pour deux expulsions consécutives desdites expulsions d'air, chaque expulsion consécutive entraînant une dose unique respective contenue dans ladite chambre à fluide (92) dans ladite chambre de mélange (95).

4. Dispositif (1) selon l'une quelconque des revendications précédentes, ladite chambre à fluide (92) étant configurée avec un volume correspondant au volume de ladite dose dudit liquide.

5. Dispositif (1) selon l'une quelconque des revendications précédentes, comprenant un activateur, tel qu'une cartouche pressurisée ou une poignée rotative (28), pour activer, de préférence en une seule opération, ledit premier entraînement et ledit deuxième entraînement, dans lequel l'activation dudit premier entraînement et dudit deuxième entraînement fournit l'énergie nécessaire pour respectivement au moins deux expulsions consécutives desdites expulsions d'air de ladite chambre à air (58) et au moins deux délivrances consécutives de ladite délivrance d'une dose à ladite chambre à fluide (92).

6. Dispositif (1) selon l'une quelconque des revendications précédentes, ledit premier entraînement comprenant :
- un ressort (61), tel qu'un ressort mécanique,
- un piston à air (55) comprenant une tête de piston (54) reçue de manière déplaçable à l'intérieur d'une structure (60), telle qu'un cylindre, définissant ladite chambre à air (58),
- ledit ressort (61) agissant sur ledit piston pneumatique (55),
- ladite excitation, ou activation dudit premier entraînement, déplaçant ledit piston à air (55) vers une position rétractée tout en comprimant ledit ressort (61),
- le dispositif (1) comprenant en outre un mécanisme de maintien et de libération (30) pour maintenir et libérer ledit piston à air (55) dans ladite position rétractée et à partir de celle-ci, pour que ledit ressort (61) entraîne ledit piston à air (55) vers une position avancée, assurant ladite expulsion.

7. Dispositif selon la revendication précédente, ledit mécanisme de maintien et de libération (30) étant configuré pour fournir une avance par étapes dudit piston à air (55) vers de multiples positions avancées.

8. Dispositif selon l'une quelconque des revendications précédentes, ledit deuxième entraînement comprenant :
- un ressort (161), tel qu'un ressort mécanique,
- un piston à liquide (110) comprenant une tête de piston (154) reçue de manière déplaçable à l'intérieur de ladite chambre de dosage (100),
- ledit ressort (161) dudit deuxième entraînement agissant sur ledit piston à liquide (110),
- ladite excitation, ou activation dudit deuxième entraînement, déplaçant ledit piston à liquide (110) vers une position rétractée tout en comprimant ledit ressort (161) et en aspirant ledit liquide dans ladite chambre de dosage (100) depuis ladite alimentation (70),
- le dispositif (1) comprenant en outre un mécanisme de maintien et de libération (130) pour maintenir et libérer ledit piston à liquide (110) dans et depuis ladite position rétractée, pour que ledit ressort (161) dudit deuxième entraînement entraîne ledit piston à liquide (110) vers une position avancée pour ladite délivrance d'une dose unique dudit fluide dans ladite chambre à fluide (92).

9. Dispositif selon la revendication précédente, ledit mécanisme de maintien et de libération (130) étant configuré pour fournir une avance par étapes dudit piston à liquide (110) vers de multiples positions avancées.

10. Dispositif selon l'une quelconque des revendications précédentes, comprenant une structure (B) avec une première partie (200), comprenant de préférence ladite buse (25), et reliée à une deuxième partie (210), ladite première partie (200) ou et / ou ladite deuxième partie (210) ayant un ou plusieurs évidements (201, 202) formés dans une surface (S) de celle-ci, lesdits évidements (201, 202) définissant au moins un conduit (301, 302) lorsque ladite première partie (200) est assemblée avec ladite deuxième partie (210), ledit conduit (301, 302) s'étendant de ladite alimentation (70) à ladite chambre de dosage (100) et / ou de ladite chambre de dosage (100) à ladite chambre à fluide (92), ledit conduit (301, 302) comprenant éventuellement des vannes unidirectionnelles (321, 322), telles que des pattes élastiques, configurées pour bloquer tout écoulement de fluide vers ladite alimentation (70) sur ledit liquide étant fourni à ladite chambre de dosage (100), ladite première partie (210) comprenant de préférence un passage (93) reliant ladite chambre à liquide (92) à ladite chambre de mélange (95) et / ou un passage (91) reliant ladite chambre à air (58) avec ladite chambre à liquide (92) et / ou un ou plusieurs passages (97) reliant séparément ladite chambre à air (58) à ladite chambre de mélange (95), lesdits un ou plusieurs passages séparés (97) s'enroulant éventuellement autour de ladite chambre à fluide (92).
